(19) Europäisches Patentamt

European Patent Office

Office européen des brevets

(11) **EP 1 242 045 B1**

(12) **FASCICULE DE BREVET EUROPEEN**

(45) Date de publication et mention
de la délivrance du brevet:
**13.09.2006 Bulletin 2006/37**

(21) Numéro de dépôt: **00988910.6**

(22) Date de dépôt: **14.12.2000**

(51) Int Cl.:
*A61K 8/97* (2006.01)   *A61Q 19/00* (2006.01)
*A61Q 5/00* (2006.01)   *A61Q 3/00* (2006.01)

(86) Numéro de dépôt international:
**PCT/FR2000/003530**

(87) Numéro de publication internationale:
**WO 2001/045648 (28.06.2001 Gazette 2001/26)**

(54) **UTILISATION D'UN EXTRAIT DU GENRE VACCINIUM COMME AGENT ANTI-GLYCATION**

VERWENDUNG EINES EXTRAKTES DER GATTUNG VACCINIUM ALS ANTI-GLYKATIONS-AGENS

USE OF AN EXTRACT OF AT LEAST ONE VACCINIUM-TYPE PLANT AS AN ANTI-GLYCATION AGENT

(84) Etats contractants désignés:
**AT BE CH CY DE DK ES FI FR GB GR IE IT LI LU MC NL PT SE TR**

(30) Priorité: **21.12.1999  FR 9916166**

(43) Date de publication de la demande:
**25.09.2002  Bulletin 2002/39**

(73) Titulaire: **L'ORÉAL**
**75008 Paris (FR)**

(72) Inventeur: **PAGEON, Hervé**
**F-92800 Puteaux (FR)**

(74) Mandataire: **Allab, Myriam et al**
**L'OREAL - D.I.P.I.**
**25-29 Quai Aulagnier**
**92600 Asnières (FR)**

(56) Documents cités:
**EP-A- 0 914 816**          **WO-A-98/51291**
**FR-A- 2 612 775**          **FR-A- 2 663 848**
**FR-A- 2 736 263**          **FR-A- 2 744 366**
**GB-A- 2 159 053**          **US-A- 5 384 123**

• **DATABASE CAPLUS [en ligne] CHEMICAL ABSTRACTS SERVICE, COLUMBUS, OHIO, US; retrieved from STN Database accession no. 2000: 658029 XP002167156 & JP 2000 256176 A (SHALOME KK) 6 juin 2000 (2000-06-06)**

**Description**

L'invention se rapporte à l'utilisation, à titre de principe actif, dans un milieu physiologiquement acceptable, d'au moins un extrait d'au moins un végétal du genre Vaccinium dans une composition ou pour la préparation d'une composition, l'extrait ou la composition étant destinés à diminuer voire inhiber la glycation des protéines, particulièrement des protéines de la peau et/ou de ses annexes.

La glycation est un processus non enzymatique faisant intervenir un ose (glucose ou ribose) qui réagit selon la réaction de Maillard avec un groupement aminé d'un résidu d'acide aminé (comme par exemple la lysine), particulièrement un résidu d'acide aminé d'une protéine, pour former une base de Schiff. Celle-ci, après un réarrangement moléculaire dit d'Amadori, peut conduire, par une succession de réactions, à un pontage, particulièrement intramoléculaire comme par exemple de type pentosidine.

Ce phénomène augmente de façon régulière avec l'âge. Il se caractérise par l'apparition de produits de glycation dont la teneur augmente de façon régulière en fonction de l'age. Les produits de glycation sont par exemple la pyrraline, la carboxyméthyl-lysine, la pentosidine, la crossline, la N$^\varepsilon$(2-carboxyéthyl)-lysine (CEL), la glyoxal-lysine dimer (GOLD), la méthylglyoxallysine dimer (MOLD), la 3DG-ARG imidazolone, les versperlysines A, B, C, la thréosidine ou encore les produits terminaux de glycosylation avancée (advanced glycosylation ends products ou AGEs).

La glycation des protéines est donc un phénomène universel, bien connu au niveau de la peau, particulièrement au niveau de sa composante dermique, mais qui survient également dans les annexes de celle-ci comme les ongles ou les cheveux, particulièrement sur les kératines et plus généralement dans tout système protéique pour peu que les conditions requises pour la glycation soient réunies.

La peau humaine est constituée de deux compartiments à savoir un compartiment superficiel, l'épiderme, et un compartiment profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même de différentes protéines extracellulaires parmi lesquelles figure notamment les fibres de collagène, l'élastine et différentes glycoprotéines. L'ensemble de ces composants extracellulaires est synthétisé par le fibroblaste. On trouve également dans le derme des leucocytes, des mastocytes ou encore des macrophages tissulaires. Enfin le derme contient des vaisseaux sanguins et des fibres nerveuses.

Le fibroblaste, de par son activité dans la synthèse des protéines extracellulaires matricielles (protéoglycannes, fibres de collagène et autres glycoprotéines de structure) est l'acteur principal de l'élaboration structurelle du derme.

Les fibres de collagène assurent la solidité du derme. Elles sont très résistantes mais sensibles à certaines enzymes appelées généralement collagénases. Dans le derme, les fibres de collagène sont constituées de fibrilles scellées les unes aux autres, formant ainsi plus de dix types de structures différentes. La structure du derme est en grande partie due à l'enchevêtrement des fibres de collagène tassées. Les fibres de collagène participent à la tonicité de la peau.

Les fibres de collagènes sont régulièrement renouvelées mais ce renouvellement diminue avec l'âge ce qui entraîne notamment un amincissement du derme. Il est également admis que des facteurs extrinsèques comme les rayons ultraviolets, le tabac ou certains traitements (Acide rétinoïque et dérivés, glucocorticoïdes, vitamine D et dérivés par exemple) ont également un effet sur la peau et sur son taux de collagène.

Au niveau de la composante dermique de la peau, la glycation intervient principalement dans le derme, sur les fibres de collagène, selon le processus décrit plus haut. La glycation du collagène augmente de façon régulière avec l'âge entraînant une augmentation régulière de la teneur de la peau en produits de glycation.

Sans vouloir introduire une quelconque théorie du vieillissement de la peau, il faut noter que d'autres modifications du collagène qui pourraient également être une conséquence de la glycation, comme une diminution de la dénaturation par la chaleur, une augmentation de la résistance à la digestion enzymatique et une augmentation des pontages intermoléculaires, ont pu être mises en évidence au cours du vieillissement de la peau (Tanaka S. et col., 1988, J. Mol. Biol., 203, 495-505 ; Takahashi M. et col., 1995, Analytical Biochemistry, 232, 158-162). De plus, des modifications dues à la glycation de certains constituants de la membrane basale comme le collagène IV, la laminine et la fibronectine ont pu être mises en évidence (Tarsio JF. et col. , 1985, Diabetes, 34, 477-484 ; Tarsio JF. et col, 1988, Diabetes, 37, 532-539 ; Sternberg M. et col., 1995, C. R. Soc. Biol., 189, 967-985).

Ainsi on comprend qu'au cours du vieillissement de la peau les propriétés physico-chimiques du collagène se modifient et ce dernier devient plus difficilement soluble et plus difficilement dégradable.

Ainsi une des composantes de la peau âgée apparaît bien être le collagène glyqué.

On sait très bien que la peau résulte d'une étroite association entre au moins deux compartiments qui la constituent à savoir l'épiderme et le derme. Les interactions entre le derme et l'épiderme sont telles qu'il est raisonnable de penser qu'une modification de l'un peut avoir des conséquences sur l'autre. On peut suspecter que le vieillissement du derme en particulier avec ses phénomènes de glycation ne peut qu'avoir des conséquences sur l'épiderme qui lui est associé. Ainsi au cours du vieillissement cutané, la glycation du collagène doit entraîner des modifications de l'épiderme qui participent nécessairement au vieillissement de l'épiderme.

Ainsi, si la glycation des protéines du derme, particulièrement du collagène entraîne autant de conséquences dommageables au niveau de la peau, des conséquences similaires sont à attendre de la glycation des protéines au niveau des annexes de la peau comme par exemple les ongles et/ou les cheveux et en fait au niveau de tout système protéique.

On comprend donc l'importance qui existe à disposer de produits qui diminuent voire inhibent le phénomène de glycation des protéines.

A cet égard la demanderesse a de manière surprenante et inattendue découvert que les extraits de végétaux du genre Vaccinium présentent la propriété de diminuer voire inhiber le phénomène de glycation des protéines.

Les végétaux du genre Vaccinium appartiennent à la famille des Ericaceae qui comprend environ une centaine de genres

Dans l'art antérieur les extraits de végétaux de la famille des Ericaceae sont utilisés entre autres comme antioxydants.

Mais, la capacité des extraits de végétaux du genre Vaccinium à diminuer voire inhiber le phénomène de glycation n'a jamais été décrite à ce jour.

L'invention a donc pour objet l'utilisation cosmétique dans une composition cosmétique d'une quantité efficace d'au moins un extrait d'au moins un végétal du genre Vaccinium, l'extrait étant destinés à diminuer voire inhiber la glycation des protéines, particulièrement la glycation des protéines de la peau et/ou de ses annexes.

Par principe actif, on entend toute molécule ou extrait susceptible de modifier ou de moduler le fonctionnement d'au moins un système biologique donné.

Très particulièrement, l'invention a pour objet l'utilisation cosmétique dans une composition cosmétique d'une quantité efficace d'au moins un extrait d'au moins un végétal du genre Vaccinium, l'extrait étant destinés à diminuer voire inhiber la glycation des protéines du derme, comme par exemple le collagène, et/ou des ongles et/ou des cheveux, comme par exemple les kératines.

Ainsi l'invention a pour objet l'utilisation cosmétique dans une composition cosmétique d'une quantité efficace d'au moins un extrait d'au moins un végétal du genre Vaccinium, l'extrait étant destinés à traiter, de manière préventive et/ou curative, les signes du vieillissement de la peau ou de ses annexes liés à la glycation.

Le genre Vaccinium, comporte plus de 450 espèces parmi lesquelles on peut citer les espèces *Vaccinium myrtillus, Vaccinium angustifollium, Vaccinium arboreum, Vaccinium arctostaphylos, Vaccinium caespitosum, Vaccinium corymbosum, Vaccinium hirsutum, Vaccinium macrocarpum, Vaccinium ovatum, Vaccinium oxycoccos, Vaccinium stamineum, Vaccinium uliginosum, Vaccinium urceolatum, Vaccinium vitis-idaea.*

Ainsi, l'extrait de végétal du genre Vaccinium de l'invention est un extrait préparé à partir de matériel issu d'au moins un végétal appartenant à une espèce choisie parmi les espèces *Vaccinium myrtillus, Vaccinium angustifollium, Vaccinium arboreum, Vaccinium arctostaphylos, Vaccinium caespitosum, Vaccinium corymbosum, Vaccinium hirsutum, Vaccinium macrocarpum, Vaccinium ovatum, Vaccinium oxycoccos, Vaccinium stamineum, Vaccinium uliginosum, Vaccinium urceolatum, Vaccinium vitis-idaea.*

Préférentiellement selon l'invention le végétal appartient à l'espèce *Vaccinium angustifollium.*

L'extrait d'au moins un végétal du genre Vaccinium peut être tout extrait préparé à partir de tout matériel végétal issu d'au moins un végétal du genre Vaccinium.

Ainsi, l'extrait d'au moins un végétal du genre Vaccinium utilisé selon l'invention peut être obtenu à partir de matériel végétal issu de plante entière ou de partie de plante comme les feuilles, les tiges, les fleurs, les pétales, les fruits, les racines ou encore des cellules dédifférenciées.

Par cellules végétales dédifférenciées, on entend toute cellule végétale ne présentant aucun des caractères d'une spécialisation particulière et capable de vivre par elle-même et non en dépendance avec d'autres cellules. Ces cellules végétales indifférenciées sont éventuellement aptes, sous l'effet d'une induction, à toute différenciation conforme à leur génome.

Selon la méthode de culture choisie, et en particulier selon le milieu de culture choisi, il est possible d'obtenir, à partir d'un même explant, des cellules végétales dédifférenciées présentant des caractères différents.

Préférentiellement selon l'invention on utilise les fruits.

L'extrait d'au moins un végétal du genre Vaccinium peut être tout extrait préparé à partir de tout matériel végétal issu d'au moins un végétal du genre Vaccinium cultivé *in vivo* ou issu de culture *in vitro.*

Par culture *in vivo* on entend toute culture de type classique c'est à dire en sol à l'air libre ou en serre, ou encore hors sol.

Par culture *in vitro,* on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo.*

Préférentiellement selon l'invention on utilise un végétal issu de culture *in vivo.*

Toute méthode d'extraction connue de l'homme du métier peut être utilisée pour préparer l'extrait contenu dans la composition selon l'invention.

On peut en particulier citer les extraits aqueux, alcooliques ou utilisant un solvant organique.

Par solvant aqueux on entend tout solvant constitué totalement ou pour part d'eau. On peut citer ainsi l'eau elle-même, les solvant hydroalcooliques en toute proportion ou encore les solvants constitués d'eau et d'un composé comme le propylène glycol en toute proportion.

Parmi les solvants alcooliques on peut citer notamment l'éthanol.

On peut également utiliser un extrait préparé par la méthode décrite dans la demande de brevet français n° 95-02379 déposée par la demanderesse.

Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape. Cette solution aqueuse correspond à l'extrait.

D'autre part, la première étape peut avantageusement être remplacée par une opération de congélation simple des tissus végétaux (par exemple à -20°C), suivie d'une extraction aqueuse reprenant les deuxièmes et troisième étapes ci-dessus décrites.

Quel que soit le mode de préparation utilisé selon l'invention des étapes subséquentes visant à favoriser la conservation et/ou la stabilisation peuvent être ajoutées sans pour cela modifier la nature même de l'extrait. Ainsi, par exemple l'extrait obtenu peut être lyophilisé par toutes méthodes classiques de lyophilisation. On obtient ainsi une poudre qui peut être utilisée directement ou bien mélanger dans un solvant approprié avant utilisation.

Préférentiellement, selon l'invention on utilise un extrait aqueux et encore plus préférentiellement un extrait réalisé avec un solvant composé d'eau et de propylène glycol, comme par exemple l'Herbasol® vendu par la société COSMETOCHEM's.

Selon l'invention les extrait d'au moins un végétal du genre Vaccinium peuvent être utilisés seuls ou en mélanges de toute nature et peuvent être d'origine naturelle ou synthétique.

Particulièrement l'extrait d'au moins un végétal du genre Vaccinium ou la composition le contenant sont utilisés selon l'invention en application topique sur la peau et/ou les ongles et/ou les cheveux.

La quantité d'extrait d'au moins un végétal du genre Vaccinium utilisable selon l'invention dépend bien évidemment de l'effet recherché et doit être en une quantité efficace pour diminuer voire inhiber la glycation.

A titre d'exemple, la quantité d'extrait d'au moins un végétal du genre Vaccinium utilisable selon l'invention peut aller par exemple de 0,001 % à 25% et de préférence de 0,005% à 15% du poids total de la composition.

En outre, la composition de l'invention est utilisée pendant un temps suffisant pour obtenir les effets attendus selon l'invention. Pour donner un ordre de grandeur, cette durée peut être au minimum de 3 semaines, mais peut être aussi de plus de 4 semaines, voire de plus de 8 semaines.

La composition est destinée à un usage cosmétique.

La composition de l'invention destinée à une application topique contient un milieu physiologiquement acceptable, c'est-à-dire compatible avec la peau y compris le cuir chevelu, ses annexes, les muqueuses et/ou les yeux et peut constituer notamment une composition cosmétique ou dermatologique.

Cette composition peut se présenter sous toutes les formes galéniques normalement utilisées dans les domaines cosmétique et dermatologique, et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles.

La composition de l'invention peut constituer par exemple une lotion, un gel, une crème ou un lait, et par exemple une lotion ou un lait de démaquillage ou de nettoyage, un shampooing ou un gel douche.

L'invention a également pour objet un procédé de traitement cosmétique pour traiter les signe du vieillissement liés à la glycation des protéines, particulièrement de la peau et/ou des ongles et/ou des cheveux, caractérisé par le fait que l'on applique sur la peau et/ou les ongles et/ou les cheveux une composition cosmétique comprenant une quantité efficace d'au moins un extrait d'au moins un végétal du genre Vaccinium, l'extrait ou la composition étant destinés à inhiber la glycation.

D'autres caractéristiques et avantages de l'invention ressortiront mieux des exemples qui suivent, donnés à titre illustratif et non limitatif. Dans ce qui suit

ou ce qui précède, les proportions sont données en pourcentage pondéral, sauf indications contraires.

Exemple 1 : Etude de l'effet d'un extrait de *Vaccinium angustifollium,* (myrtille) sur la glycation.

Une solution de sérum albumine bovine à 5 mg/ml en solution dans du tampon phosphate salin (PBS) est incubée à 37°C pendant 28 jours en présence ou en absence de D-ribose à la concentration 10mM ou d'un extrait de myrtille aux concentrations de 5% et 10%.

La glycation est évaluée en mesurant la fluorescence (Rx) des AGEs (ensemble des produits de glycation) à λem. = 440 nm émise par chaque échantillon après excitation à λex. = 370 nm ou encore à λem. = 380 nm émise par chaque échantillon après excitation à λex. = 320 nm (fluorescence émise par certain des produits de glycation dont en particulier la pentosidine).

L'inhibition de la glycation est visualisée par la diminution de la fluorescence comparée à l'échantillon traité avec le sucre seul (R) selon la formule:

$$(R-Rx)/R \times 100$$

Les résultats sont en % d'inhibition :

|  | Extrait à 5% | Extrait à 10% |
|---|---|---|
| λem. = 440 nm / λex. = 370 nm | 63 | 71 |
| λem. = 380 nm / λex. = 320 nm | 16 | 43 |

L'extrait de myrtille présente un effet anti-glycation intéressant dès la concentration de 5%.

Exemple 2 : Exemples de formulations illustrant l'invention et particulièrement les compositions selon l'invention. Ces compositions ont été obtenues par simple mélange des différents composants.

Composition 1 : Lotion

| | |
|---|---|
| Herbasol® (extrait de *Vaccinium angustifollium*) | 1,00 |
| Antioxydant | 0,05 |
| Isopropanol | 40,00 |
| Conservateur | 0,30 |
| Eau | qsp 100 % |

Composition 2 : Gel pour le soin

| | |
|---|---|
| Herbasol® (extrait de *Vaccinium angustifollium*) | 4,00 |
| Hydroxypropylcellulose* | 1,00 |
| Antioxydant | 0,05 |
| Isopropanol | 40,00 |
| Conservateur | 0,30 |
| Eau | qsp 100 % |

Composition 3 : Crème de soin (émulsion huile dans eau)

| | |
|---|---|
| Herbasol® (extrait de *Vaccinium angustifollium*) | 5,00 |
| Stéarate de glycérol | 2,00 |
| Polysorbate 60** | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

Composition 4 : Shampooing

| | |
|---|---:|
| Herbasol® (extrait de *Vaccinium angustifollium*) | 2,00 |
| Hydroxypropylcellulose* | 1,00 |
| Lauryl sulfate de sodium | 12,00 |
| Parfum | 0,50 |
| Conservateur | 0,30 |
| Eau | qsp 100 % |

Composition 5 : Crème de soin (émulsion huile/eau)

| | |
|---|---:|
| Herbasol® (extrait de *Vaccinium angustifollium*) | 4,00 |
| Stéarate de glycérol | 2,00 |
| Polysorbate 60** | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

Composition 6 : Gel

| | |
|---|---:|
| Herbasol® (extrait de *Vaccinium angustifollium*) | 10,00 |
| Hydroxypropylcellulose* | 1,00 |
| Antioxydant | 0,05 |
| Chlorhydrate de lidocaïne | 2,00 |
| Isopropanol | 40,00 |
| Conservateur | 0,30 |
| Eau | qsp 100 % |

Composition 7 : Crème de soin (émulsion huile-dans-eau)

| | |
|---|---:|
| Herbasol® (extrait de *Vaccinium angustifollium*) | 20,00 |
| Stéarate de glycérol | 2,00 |
| Polysorbate 60** | 1,00 |
| Acide stéarique - | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,5 |
| Conservateur | 0,30 |
| Eau | qsp 100 % |

Composition 8 : Gel

| | |
|---|---|
| Herbasol® (extrait de *Vaccinium angustifollium)* | 8,00 |
| Acide tout trans rétinoïque | 0,05 |
| Hydroxypropylcellulose* | 1,00 |
| Antioxydant | 0,05 |
| Isopropanol | 40,00 |
| Conservateur | 0,30 |
| Eau | qsp 100 % |

\* : Klucel H® vendu par la société Hercules

\*\* : Tween 60® vendu par la société ICI

**Revendications**

1. Utilisation cosmétique dans une composition cosmétique d'une quantité efficace d'au moins un extrait d'au moins un végétal du genre Vaccinium, l'extrait étant destiné à diminuer la glycation des protéines de la peau et/ou des ongles et/ou des cheveux.

2. Utilisation selon la revendication précédente, **caractérisée par le fait que** les protéines de la peau sont les protéines du derme.

3. Utilisation selon la revendication précédente, pour diminuer la glycation du collagène.

4. Utilisation selon la revendication 1, **caractérisée par le fait que** les protéines des ongles et/ou des cheveux sont les kératines.

5. Utilisation selon l'une quelconque des revendications précédentes pour traiter, de manière préventive et/ou curative, les signes du vieillissement de la peau et/ou de ses annexes liés à la glycation.

6. Utilisation selon l'une quelconque des revendications précédentes, **caractérisée par le fait que** le végétal du genre Vaccinium est d'une espèce choisie parmi les espèces *Vaccinium myrtillus, Vaccinium angustifollium, Vaccinium arboreum, Vaccinium arctostaphylos, Vaccinium caespitosum, Vaccinium corymbosum, Vaccinium hirsutum, Vaccinium macrocarpum, Vaccinium ovatum, Vaccinium oxycoccos, Vaccinium stamineum, Vaccinium uliginosum, Vaccinium urceotatum, Vaccinium vitis-idaea.*

7. Utilisation selon la revendication précédente , **caractérisée par le fait que** le végétal du genre Vaccinium est de l'espèce *Vaccinium angustifollium.*

8. Utilisation selon l'une quelconque des revendications précédentes, **caractérisée par le fait que** l'extrait d'au moins un végétal du genre Vaccinium est présent en une quantité allant de 0,001% à 25% du poids total de la composition

9. Utilisation selon la revendication précédente, **caractérisée par le fait que** l'extrait d'au moins un végétal du genre Vaccinium est présent en une quantité allant de 0,005% à 15% du poids total de la composition.

10. Procédé de traitement cosmétique pour traiter les signes du vieillissement liés à la glycation des protéines de la peau et/ou des ongles et/ou des cheveux, **caractérisé par le fait que** l'on applique sur la peau et/ou les ongles et/ou les cheveux une composition cosmétique comprenant une quantité efficace d'au moins un extrait d'au moins un végétal du genre Vaccinium, l'extrait ou la composition étant destinés à inhiber la glycation.

**Claims**

1. Cosmetic use, in a cosmetic composition, of an effective quantity of at least one extract of at least one plant of the genus Vaccinium, the extract being intended for reducing glycation of the proteins of the skin and/or the nails and/or the hair.

**2.** Use according to the preceding claim, **characterized in that** the proteins of the skin are proteins of the dermis.

**3.** Use according to the preceding claim, for reducing the glycation of collagen.

**4.** Use according to Claim 1, **characterized in that** the proteins of the nails and/or the hair are keratins.

**5.** Use according to any one of the preceding claims, for treating, preventively and/or curatively, the signs of ageing of the skin and/or of its annexes linked to glycation.

**6.** Use according to any one of the preceding claims, **characterized in that** the plant of the genus Vaccinium is of a species chosen from the species *Vaccinium myrtillus, Vaccinium angustifollium, Vaccinium arboreum, Vaccinium arctostaphylos, Vaccinium caespitosum, vaccinium corymbosum, Vaccinium hirsutum, Vaccinium macrocarpum, Vaccinium ovatum, Vaccinium oxycoccos, Vaccinium stamineum, Vaccinium uliginosum, Vaccinium urceolatum* and *Vaccinium vitis-idaea.*

**7.** Use according to the preceding claim, **characterized in that** the plant of the genus Vaccinium is of the species *Vaccinium angustifollium.*

**8.** Use according to any one of the preceding claims, **characterized in that** the extract of at least one plant of the genus Vaccinium is present in a quantity ranging from 0.001% to 25% of the total weight of the composition.

**9.** Use according to the preceding claim, **characterized in that** the extract of at least one plant of the genus Vaccinium is present in a quantity ranging from 0.005% to 15% of the total weight of the composition.

**10.** Method of cosmetic treatment for treating the signs of ageing linked to the glycation of proteins of the skin and/or the nails and/or the hair, **characterized in that** there is applied to the skin and/or the nails and/or the hair a cosmetic composition comprising an effective quantity of at least one extract of at least one plant of the genus Vaccinium, the extract or the composition being intended for inhibiting glycation.

**Patentansprüche**

**1.** Kosmetische Verwendung einer wirksamen Menge mindestens eines Extrakts mindestens einer Pflanze der Gattung Vaccinium in einer kosmetischen Zusammensetzung, wobei der Extrakt dazu vorgesehen ist, die Glykation von Proteinen der Haut und/oder der Nägel und/oder Haare zu vermindern.

**2.** Verwendung nach dem vorhergehenden Anspruch, **dadurch gekennzeichnet, dass** die Proteine der Haut Proteine der Dermis sind.

**3.** Verwendung nach dem vorhergehenden Anspruch, um die Glykation von Collagen zu vermindern.

**4.** Verwendung nach Anspruch 1, **dadurch gekennzeichnet, dass** die Proteine der Nägel und/oder der Haare Keratine sind.

**5.** Verwendung nach einem der vorhergehenden Ansprüche, um präventiv und/oder kurativ die Anzeichen der Alterung der Haut und/oder der Hautanhangsgebilde zu behandeln, die mit der Glykation zusammenhängen.

**6.** Verwendung nach einem der vorhergehenden Ansprüche, **dadurch gekennzeichnet, dass** die Pflanze der Gattung Vaccinium eine Art ist, die unter den folgenden Arten ausgewählt ist: *Vaccinium myrtillus, Vaccinium angustifollium, Vaccinium arboreum, Vaccinium arctostaphylos, Vaccinium caespitosum, Vaccinium corymbosum, Vaccinium hirsutum, Vaccinium macrocarpum, Vaccinium ovatum, Vaccinium oxycoccos, Vaccinium stamineum, Vaccinium uliginosum, Vaccinium urceolatum, Vaccinium vitis-idaea.*

**7.** Verwendung nach dem vorhergehenden Anspruch, **dadurch gekennzeichnet, dass** die Pflanze der Gattung Vaccinium die Art *Vaccinium angustifollium* ist.

**8.** Verwendung nach einem der vorhergehenden Ansprüche, **dadurch gekennzeichnet, dass** der Extrakt mindestens einer Pflanze der Gattung Vaccinium in einer Menge von 0,001 bis 25 % des Gesamtgewichts der Zusammensetzung enthalten ist.

9. Verwendung nach dem vorhergehenden Anspruch, **dadurch gekennzeichnet, dass** der Extrakt mindestens einer Pflanze der Gattung Vaccinium in einer Menge von 0,005 bis 15 % des Gesamtgewichts der Zusammensetzung enthalten ist.

10. Verfahren zur kosmetischen Behandlung, um die Zeichen der Alterung zu behandeln, die mit der Glykation von Proteinen der Haut und/oder der Nägel und/oder der Haare zusammenhängen, **dadurch gekennzeichnet, dass** auf die Haut und/oder die Nägel und/oder die Haare eine kosmetische Zusammensetzung aufgetragen wird, die eine wirksame Menge mindestens eines Extrakts mindestens einer Pflanze der Gattung Vaccinium enthält, wobei der Extrakt oder die Zusammensetzung zur Inhibierung der Glykation dienen sollen.